# EUROPEAN PATENT SPECIFICATION

(11) **EP 2 093 205 B1**
(45) Date of publication and mention of the grant of the patent: **02.03.2011**
(21) Application number: 08164335.5
(22) Date of filing: 15.09.2008
(51) Int. Cl.: C07C 5/48, C07C 5/42

(54) **Catalytic Process For Producing Ethylene And Carbon Monoxide Mixtures From Ethane**
Katalytisches Verfahren zur Herstellung von Ethylen- und Kohlenmonoxid-Gemischen aus Ethan
Procédé catalytique pour la production de mélanges d'éthylène et de monoxyde de carbone à base d'éthane

(30) Priority: 20.09.2007 US 994641 P
(43) Date of publication of application: 26.08.2009
(73) Proprietor: Rohm and Haas Company, Philadelphia, PA 19106-2399 (US)
(72) Inventor: Banavali, Rajiv Manohar, Rydal, PA 19046 (US); Benderly, Abraham, Elkins Park, PA 19027 (US); Han, Scott, Lawrenceville, NJ 08648 (US); Martenak, Daniel Joseph, Perkasie, PA 18944 (US); Trejo, Jose Antonio, Lansdale, PA 19446 (US)
(74) Representative: Kent, Venetia Katherine

(56) References cited:
- US-A- 2 604 495
- US-A- 4 408 079
- US-B1- 6 284 919
- ZHAO X ET AL: "Oxidative dehydrogenation of ethane to ethylene by carbon dioxide over Cr/TS-1 catalysts" CATALYSIS COMMUNICATIONS, ELSEVIER SCIENCE, AMSTERDAM, NL, vol. 7, no. 9, 1 September 2006 (2006-09-01), pages 633-638, XP024973044 ISSN: 1566-7367 [retrieved on 2006-09-01]
- KRYLOV O V ET AL: "Catalytic Oxidation of Hydrocarbons and Alcohols by Carbon Dioxide on Oxide Catalysts" INDUSTRIAL & ENGINEERING CHEMISTRY RESEARCH, AMERICAN CHEMICAL SOCIETY, US, vol. 34, no. 2, 1 January 1995 (1995-01-01), pages 474-482, XP002510987 ISSN: 0888-5885

## Description

The present invention relates to a catalytic process for producing ethylene and carbon monoxide mixtures from ethane and carbon dioxide, and further to various integrated processes for producing alkyl propionates or methacrylic acid esters from ethane and carbon dioxide.

Ethylene and carbon monoxide mixtures are used as a feedstock for homologation of ethylene to propionic acid derivatives. For example, carbonylation of ethylene to produce methyl propionate, followed by condensation with formaldehyde, is an important commercial route to methyl methacrylate. For example, U.S. Pat. No. 6,284,919 discloses a process for carbonylation of ethylene to methyl propionate. In the first step of this process, ethylene, CO, and methanol feed is converted to methyl propionate. The ethylene and CO feeds used would generally be from conventional sources such as steam cracking and methane steam reforming. However, due to the high cost associated with these ethylene-producing processes, ethylene is a relatively expensive starting material. A process that uses ethane, which is a component of natural gas, as a starting material would be economically desirable due to the large price difference between ethane and ethylene. An integrated process which provides for the production of esters such as methyl propionate and methyl methacrylate using cheap and abundantly available feeds would be of high value. In addition, ethylene and carbon monoxide feeds often are combined with hydrogen in the industrially important hydroformylation reaction. This reaction, also known as the oxo reaction, is used mainly for conversion of olefins to aldehydes and alcohols.

The problem addressed by this invention is to provide an alternative catalytic process for producing mixtures of ethylene and carbon monoxide suitable as a feedstock for other processes.

U.S. Patent No. 2,604,495 discloses the conversion of ethane and carbon dioxide to ethylene and carbon monoxide at a temperature above 600°C using a catalyst which comprises iron oxide, but which is not supported on an inorganic material comprising calcium oxide or calcium carbonate.

### STATEMENT OF THE INVENTION

The present invention provides a method for producing a mixture of ethylene and carbon monoxide by contacting ethane and carbon dioxide with a catalyst comprising iron oxide supported on an inorganic material comprising at least one of calcium oxide and calcium carbonate at a temperature of at least 600°C to produce ethylene and carbon monoxide.

The present invention further comprises steps of: (a) contacting an alcohol, and the ethylene and carbon monoxide with an ethylene carbonylation catalyst to produce an alkyl propionate; and (b) separating the alkyl propionate from byproducts and starting materials, thus providing an integrated process for producing an alkyl propionate. The invention further comprises condensing the alkyl propionate with formaldehyde, thus providing an integrated process for producing methacrylic acid esters. The invention further comprises converting the ethylene and carbon monoxide to copolymers. The invention further comprises combining the ethylene and carbon monoxide with hydrogen to produce propionaldehyde, and optionally, condensing propionaldehyde with formaldehyde to produce methacrolein.

### DETAILED DESCRIPTION OF THE INVENTION

Percentages are weight percentages ("wt %"), and temperatures are in °C, unless specified otherwise. An alkyl group is a saturated hydrocarbyl group having from one to twenty carbon atoms, and may be linear or branched. Preferably, alkyl groups have from one to eight carbon atoms, alternatively from one to four carbon atoms, alternatively one or two carbon atoms, alternatively one carbon atom. The alcohol used in the ethylene carbonylation reaction corresponds to an alkyl group, as defined above, substituted with a hydroxyl group. An "inorganic material" is one containing at most trace levels, e.g., no more than 0.5%, of carbon compounds other than metal carbonates.

In some embodiments of the invention, the inorganic material comprising at least one of calcium oxide and calcium carbonate is derived from calcium carbonate materials containing at least 50% calcium carbonate, alternatively at least 85% calcium carbonate; in some embodiments, the maximum calcium carbonate content is 97%. Particles of the calcium carbonate material are combined with an iron compound, resulting in iron-coated particles. In some embodiments of the invention, the calcium carbonate material is treated with an iron salt and then calcined to produce iron oxides, preferably iron(III) oxides. In some embodiments of the invention, the calcium carbonate material is coated with hydrous iron oxides and then heated under calcining conditions. In the catalyst, the calcium carbonate in these materials may be at least partially converted to calcium oxide by the calcination process and/or by the reaction conditions for converting ethane to ethylene, depending on the temperatures to which the catalyst is exposed. Examples of materials containing calcium carbonate include, e.g., limestone, aragonite, vaterite, marble, dolomite and coral. These minerals often contain variable amounts of silica, clay, silt and sand; and they may be crystalline, clastic, granular or massive, depending on the method of formation. Crystals of calcite, quartz, dolomite or barite may line small cavities in the rock. In some embodiments of the invention, the calcium carbonate material is limestone powder having an average particle size from 10 to 100 microns, alternatively from 30 to 70 microns. In some embodiments, the material comprises limestone chips, preferably in a range from 100 microns to 10 mm, alternatively from 100 to 800 microns.

The term "hydrous oxide" or "oxyhydroxide" refers to a mixture of iron compounds precipitated from water at high pH. Hydrous oxides may be oxides and/or hydroxides of iron. Their structure may be amorphous or crystalline. Examples of iron oxides useful in the present invention include, e.g., amorphous and crystalline forms including goethite, lepidocrocite, schwertmannite, feroxhyte, ferrihydrite, hematite, magnetite, maghemite, wustite, bernalite and green rusts. Typically, hydrous oxides of iron are formed by increasing the pH of an aqueous iron salt above 3, preferably from 5 to 7, by addition of aqueous base. In some embodiments of the invention, the aqueous iron salt is an aqueous ferric ion salt, e.g. the chloride, preferably a 40% w/v solution. Particles of material containing calcium carbonate can be coated with hydrous oxides by adding base to a reactor containing the particles and an aqueous solution of an iron salt at a pH below 3. The average thickness of the coating is from 5 to 50 microns. The coverage of the material containing calcium carbonate can be complete or partial. In some embodiments of the invention, the surface area of the iron-coated particles is from 20 to 80 m²/g, and the pore size is 20 to 50 Å, as measured by BET porosimetry. In some embodiments of the invention, excess water is drained from the coated particles, and they are dried to achieve a moisture content from 2 to 30%, alternatively from 2 to 15%. In some embodiments of the invention, a soluble iron salt is added to the calcium carbonate material using the incipient wetness technique, in which aqueous iron salt is added just to the point of saturating the particles of solid calcium carbonate material. Iron salts useful in the incipient wetness technique are water-soluble iron salts, i.e., those having at least 10% solubility in water (w/v), including, e.g., ferric nitrate, ferric sulfate and ferric chloride. In some embodiments of the invention, the calcium carbonate material is contacted with a solution of a ferrous (iron(II)) salt, and then with an oxidizing agent to produce a precipitate of hydrous iron (III) oxides on the calcium carbonate material. Suitable oxidizing agents include, e.g., peroxides, permanganate and manganese dioxide. Materials made with the incipient wetness or oxidation techniques are calcined to produce the catalyst.

Preferably, the iron content of the iron-coated particles, on a dry basis, is from 1% to 50%. In some embodiments of the invention, the iron content is at least 2%, alternatively at least 3%, alternatively at least 4%. In some embodiments, the iron content is no more than 25%, alternatively no more than 15%, alternatively no more than 10%, alternatively no more than 8%.

Preferably, the iron-coated particles are calcined at a temperature of at least 275°C, alternatively at least 400°C, alternatively at least 500°C, alternatively at least 550°C, alternatively at least 600°C; preferably the temperature is no greater than 1000°C, alternatively no greater than 900°C, alternatively no greater than 850°C. Preferably, the calcination is performed in a stream of inert gas containing oxygen, e.g., a stream of nitrogen containing 5% to 25% oxygen, including, e.g., an air stream. The calcination time preferably is at least 15 minutes, alternatively at least 30 minutes, alternatively at least 1 hour; preferably the time is no greater than 5 hours, alternatively no greater than 3 hours.

In some embodiments of the invention, the catalyst, i.e., the calcined calcium carbonate material containing iron salts, contains from 1% to 50% iron. In some embodiments of the invention, the iron content is at least 2%, alternatively at least 3%, alternatively at least 4%. In some embodiments, the iron content is no more than 25%, alternatively no more than 15%, alternatively no more than 10%, alternatively no more than 8%, alternatively no more than 6%.

Preferably, the ethane and carbon dioxide are contacted with the catalyst at a temperature of at least 600°C, alternatively at least 650°C, alternatively at least 700°C, alternatively at least 750°C. Preferably, the ethane and carbon dioxide are contacted with the catalyst at a temperature no greater than 1000°C, alternatively no greater than 950°C, alternatively no greater than 900°C, alternatively no greater than 875°C. More than one catalyst may be present. In some embodiments of the invention, the only catalysts present are those of the type described herein. Preferably, the flow rate of the feed is from 25 to 500 cm³/min, alternatively from 50 to 300 cm³/min, alternatively from 50 to 200 cm³/min. Preferably, the molar ratio of carbon dioxide to ethane in the feed is from 1:1 to 10:1, alternatively from 1:1 to 4:1, alternatively from 1.5:1 to 3:1.

In addition to ethane and carbon dioxide, inert carrier gases may be present, e.g., nitrogen. Inert carriers do not participate in, and are unaffected by, the reactions of concern.

Ethylene carbonylation catalysts and conditions are well known, and are described, e.g., in U.S. Pat. No. 6,284,919. Typical catalysts include, e.g., those having a Group VIII metal, e.g. palladium, and a phosphine ligand, e.g. an alkyl phosphine, cycloalkyl phosphine, aryl phosphine, pyridyl phosphine or bidentate phosphine.

In some embodiments of the invention, the products of reaction of ethane and carbon dioxide, which comprise ethylene and carbon monoxide, are contacted with an ethylene carbonylation catalyst, along with an alcohol. The ethylene and carbon monoxide stream may be passed into a different reactor for carbonylation, or alternatively, into another portion of the same reactor. The alkyl propionate product can be converted to an alkyl acrylate in an oxidative dehydrogenation process.

Unreacted ethane and carbon dioxide may be present in the product stream from reaction of ethane and carbon dioxide, as well as in the product stream from carbonylation. After separation of the carbonylation product stream, ethane and carbon oxides may be recycled to the input of the reaction of ethane and carbon dioxide. Trace amounts of ethylene and alcohol may also be present. Unreacted ethylene and alcohol from the carbonylation reaction may be recycled to the input of the carbonylation reaction.

In some embodiments of the invention, the alcohol is methanol, the alkyl propionate is methyl propionate and the alkyl methacrylate is methyl methacrylate. In these embodiments, the method represents an integrated process for producing methyl methacrylate starting from ethane and carbon dioxide.

In some embodiments of the invention, the ethylene and carbon monoxide products from the reaction of ethane and carbon dioxide are subjected to a hydroformylation reaction to produce propionaldehyde, as described, e.g., in U.S. Pat. No. 4,408,079. The propionaldehyde product can be oxidized to propionic acid or condensed with formaldehyde to produce methacrolein, which in turn can be used to produce methacrylic acid.

In some embodiments of the invention, the method further comprises polymerization of the methyl methacrylate product to provide an integrated process for producing methyl methacrylate polymers or copolymers starting from ethane and carbon dioxide.

In some embodiments of the invention, methanol is used to produce methyl methacrylate as described herein, and the methyl methacrylate then is transesterified with other alcohols to produce other alkyl methacrylates.

In some embodiments of the invention, the ethylene and carbon monoxide are copolymerized. Preferably, a palladium compound is used as a catalyst, e.g., palladium cyanide, aryl phosphine complexes of palladium or palladium halides, or tetrakis triarylphosphine platinum complex. Polymerization processes are described, e.g., in U.S. Pat. Nos. 3,530,109 and 3,694,412. The ethylene-carbon monoxide polymer can be converted to a thermosetting compound by heating.

In some embodiments of the invention, ethane, carbon dioxide and oxygen are reacted under millisecond contact times resulting in an autothermal reaction. Millisecond contact times are times less than one second, alternatively less than 900 milliseconds, alternatively less than 500 milliseconds, alternatively less than 100 milliseconds, alternatively less than 50 milliseconds, alternatively less than 10 milliseconds. In some embodiments of the invention, ethane and carbon dioxide react either in a single reactor or in staged reactors to provide improved heat balance.

### EXAMPLES

Catalysts were produced by precipitating hydrous iron oxides on limestone particles from a 40% w/v solution of ferric chloride. The solution was allowed to stand in the presence of the limestone prior to base addition. Precipitation occurred on addition of 10% NaOH to a pH of 5-7. The coated substrate was then calcined at 300°C in a stream of 90% nitrogen/10% oxygen for one hour. The catalyst preparation amounts and characteristics were as follows:

| particle size, µm | limestone, g | water, g | FeCl₃ solution, mL | min. < base addition | 10% NaOH, mL | pH | water wash, mL | %Fe, dry basis |
|---|---|---|---|---|---|---|---|---|
| | | | | | | | | |
| 425-710 | 130 | 130 | 100 | 80 | 75 | 7.02 | 5400 | 4.5 |
| 425-710 | 130 | 130 | 260 | 20 | 225 | 6.61 | 6500 | 5.4 |

A catalyst was prepared by treating CaCO₃ (20g, ACS reagent, 99%⁺ purity, measured pore volume of ∼0.60cm³g⁻¹) with 6ml of a homogeneous solution of iron (III) nitrate (nona hydrate, ∼13wt% Fe). The incipient wetness process was carried out at room temperature by dropwise addition of the iron nitrate solution with constant mixing (manual stirring followed by Vortex mixer for 30 min) to ensure uniformity.

After 30 min of soaking in closed containment, the resulting brown paste-like precursor was placed in a ceramic dish, and the large chunks were pulverized to 2-3 mm size grains. The mixture was then calcined in a programmable isothermal box furnace as follows: 4 hr at 80°C then drying at 120°C for 4 hr, followed by a calcination step carried out at 300°C for 2 hr with continuous air-purge (5 SLPM). The catalyst was then charged into the reactor for additional heat treatment prior to the catalytic evaluation step. This calcination step took place at 600°C for one hour while a gaseous stream consisting of 10% O₂ and 90% N₂ was passing at 100 cm³/min over the catalyst bed.

Catalytic experiments were carried out using 4 mL of the catalyst diluted with 4 mL of silicon carbide chips charged to a ½" (12.7 mm) O.D. stainless steel reactor tube. The reactor was heated to the desired temperature in flowing N₂. Once at temperature, a feed comprising CO₂:C₂H₆:N₂ in a predetermined molar ratio, as noted in Table 1, was introduced into the reactor. The gases were fed at 100 mL/min total. Analysis of the products was by GC and N₂ was employed as an internal standard. Feed conversions and product yields were calculated on a molar basis. Data obtained from the experiment described above are presented in Table 1 ("sel" is selectivity, "conv" is conversion). The data demonstrate that changes in catalyst, temperature and feed affect the ethylene to carbon monoxide ratio, and that a ratio near 1:1 can be obtained, which is optimal for carbonylation and other reactions of ethylene and carbon monoxide.

**Table 1. Process conditions and data for ethane/CO₂ conversion with Fe-based catalysts.**

| Example | Catalyst*** %Fe/substrate | Feed flow cc/min | Res. time sec | Rxn. temp °C | C₂H₆ conv mol% | CO₂ conv mol% | C₂H₄ sel* mol% | CO sel** mol% | C₂H₄ yield* mol% | CO yield** mol% | C₂H₄:CO ratio mol/mol |
|---|---|---|---|---|---|---|---|---|---|---|---|
| | | | | | | | | | | | |

| Feed is CO₂:C₂H₆:N₂ in molar ratio of 3:1:1 | | | | | | | | | | | |
|---|---|---|---|---|---|---|---|---|---|---|---|
| | | | | | | | | | | | |
| 1 | 5.4/limestone | 100 | 1.2 | 795 | 52.4 | 12.9 | 95.3 | 98.3 | 49.9 | 12.7 | 1.32 |
| | | | | | | | | | | | |
| 2 | 5.4/limestone | 100 | 1.8 | 797 | 57.4 | 10.9 | 90.5 | 109.7 | 52.0 | 11.9 | 1.45 |
| | | | | | | | | | | | |
| 3 | 4.5/limestone | 100 | 3.6 | 791 | 44.7 | 10.4 | 100.0 | 139.3 | 45.1 | 14.5 | 1.04 |
| | | | | | | | | | | | |

| Feed is CO₂:C₂H₆:N₂ in molar ratio of 55:30:15 | | | | | | | | | | | |
|---|---|---|---|---|---|---|---|---|---|---|---|
| | | | | | | | | | | | |
| 4 | 4.5/CaCO₃ | 100 | 3.6 | 800 | 53.9 | 22.3 | 95.2 | 97.8 | 51.2 | 21.8 | 1.28 |
| | | 100 | 3.6 | 800 | 55.8 | 23.3 | 91.2 | 93.3 | 50.9 | 21.7 | 1.28 |
| | | 100 | 3.6 | 800 | 56.6 | 24.0 | 89.5 | 90.4 | 50.6 | 21.7 | 1.28 |
| | | 100 | 3.6 | 800 | 56.2 | 23.2 | 90.6 | 90.0 | 50.4 | 21.3 | 1.28 |
| | | 100 | 3.6 | 800 | 56.7 | 23.7 | 88.9 | 89.9 | 50.6 | 21.3 | 1.29 |
| | | | | | | | | | | | |
| 5 | 4.5/CaCO₃ | 100 | 3.6 | 820 | 71.5 | 31.0 | 83.8 | 99.3 | 59.9 | 30.7 | 1.06 |
| | | 100 | 3.6 | 820 | 71.4 | 31.0 | 84.1 | 99.8 | 60.1 | 30.9 | 1.06 |
| | | 100 | 3.6 | 820 | 71.5 | 31.6 | 83.6 | 97.1 | 59.7 | 30.7 | 1.06 |
| | | 100 | 3.6 | 820 | 71.4 | 31.5 | 83.4 | 96.8 | 59.5 | 30.5 | 1.06 |
| | | 100 | 3.6 | 820 | 71.7 | 31.6 | 82.5 | 95.7 | 59.2 | 30.2 | 1.07 |
| | | 100 | 3.6 | | 820 71.5 | 31.3 | 82.9 | 96.3 | 59.3 | 30.2 | 1.07 |
| | | | | | | | | | | | |
| | | | | | | | | | | | |
| Feed is CO₂:C₂H₆:N₂ in molar ratio of 45:40:15 | | | | | | | | | | | |
| | | | | | | | | | | | |
| | 4.5/CaCO₃ | 100 | 3.6 | 800 | 54.2 | 28.8 | 86.9 | 100.9 | 47.1 | 29.1 | 1.44 |
| | | 100 | 3.6 | 800 | 56.0 | 28.6 | 85.8 | 99.8 | 48.1 | 28.6 | 1.49 |
| | | 100 | 3.6 | 800 | 56.4 | 28.4 | 85.1 | 98.5 | 48.0 | 28.0 | 1.53 |
| | | | | | | | | | | | |
| *Based on C₂H₆ conv. | | | | | | | | | | | |
| **Based on CO₂ conv. | | | | | | | | | | | |
| ***Fe level prior to calcination | | | | | | | | | | | |

### Preparation of FeOx/CaCO₃ catalyst by incipient wetness (IW) method

A commercial sample of CaCO₃ (20g, ACS reagent, 99%⁺ purity, measured pore volume of ∼0.60cm³g⁻¹) was treated with 6 mL of a homogeneous solution of Fe(III) nitrate (nona-hydrate ∼13wt% Fe). The incipient wetness process was carried out at room temperature by dropwise addition of the iron nitrate solution with constant mixing (manual stirring followed by Vortex mixer for 30 min) to ensure uniformity).

After 30 min of soaking in closed containment, the resulting brown paste-like precursor was placed in a ceramic dish, and the large chunks were pulverized to 2-3 mm size grains. The mixture was then calcined in a programmable isothermally box furnace as follows: 4 hr at 80°C then drying at 120°C for 4 hr, followed by a calcination step carried out at 300°C for 2 hr with continuous air-purge (5 SLPM). The catalyst was then charged into the reactor for additional heat treatment prior to the catalytic evaluation step. This calcination step took place at 600°C for one hour while a gaseous stream consisting of 10% O₂ and 90% N₂ was passing at 100 cm³/min over the catalyst bed. XRF analysis of the calcined catalyst gave the following results for wt% of various metals:

| Ca | Fe | Si | Na | Sr | Mg | Cr |
|---|---|---|---|---|---|---|
| 35.7 | 4.1 | 0.08 | 0.09 | 0.03 | 0.01 | 0.01 |

ODE process with the catalyst FeOx/CaCO₃ prepared by (IW) method

(CO₂:C₂H₆:N₂ molar ratio of 55:30:15, flow rate 100 mL/min, residence time 1.8 s)

| T°C | TOS (hr) | C₂ | C₂⁼ | | CO | C₂⁼ /CO | CO₂ |
|---|---|---|---|---|---|---|---|
| | | %C | %S | %Y | %Y | | %C |
| 800 | 0.5 | 63.4 | 85.0 | 53.9 | 27.2 | 1.08 | 30.6 |
| 800 | 1.75 | 67.8 | 80.4 | 54.5 | 25.5 | 1.17 | 30.8 |
| 800 | 2.5 | 67.6 | 81.6 | 55.1 | 26.0 | 1.15 | 30.9 |
| 810 | 4.75 | 73.6 | 79.0 | 58.1 | 38.7 | 0.82 | 35.9 |

| | | | | | | | |
|---|---|---|---|---|---|---|---|
| C=conversion, S=selectivity, Y=yield, TOS=catalyst time on stream | | | | | | | |

## Claims

1. A method for producing a mixture of ethylene and carbon monoxide by contacting ethane and carbon dioxide with a catalyst comprising iron oxide supported on an inorganic material comprising at least one of calcium oxide and calcium carbonate at a temperature of at least 600°C to produce ethylene and carbon monoxide.

2. The method of claim 1 in which the catalyst contains from 2% to 25% iron.

3. The method of claim 2 in which the catalyst is produced by coating particles of a material containing at least 50% calcium carbonate with hydrous oxides of iron, and then calcining at a temperature of at least 275°C.

4. The method of claim 1, further comprising steps of:
(a) contacting an alcohol, and said ethylene and carbon monoxide with an ethylene carbonylation catalyst to produce an alkyl propionate; and
(b) separating the alkyl propionate from byproducts and starting materials.

5. The method of claim 4, further comprising reacting the alkyl propionate with formaldehyde to produce an alkyl methacrylate.

6. The method of claim 5 in which the alcohol is methanol, the alkyl propionate is methyl propionate and the alkyl methacrylate is methyl methacrylate.

7. The method of claim 6, further comprising polymerizing the methyl methacrylate.

8. The method of claim 1, further comprising co-polymerizing the ethylene and carbon monoxide.

9. The method of claim 1, further comprising combining said ethylene and carbon monoxide with hydrogen to produce propionaldehyde.

10. The method of claim 9, further comprising condensing the propionaldehyde with formaldehyde to produce methacrolein.

## Patentansprüche

1. Verfahren zum Herstellen eines Gemischs aus Ethylen und Kohlenmonoxid durch Inkontaktbringen von Ethan und Kohlendioxid mit einem Katalysator, umfassend Eisenoxid, getragen auf einem anorganischen Material, umfassend mindestens eines von Calciumoxid und Calciumcarbonat, bei einer Temperatur von mindestens 600°C, um Ethylen und Kohlenmonoxid herzustellen.

2. Verfahren nach Anspruch 1, in welchem der Katalysator 2% bis 25% Eisen enthält.

3. Verfahren nach Anspruch 2, in welchem der Katalysator hergestellt wird durch Beschichten von Teilchen eines Materials, enthaltend mindestens 50% Calciumcarbonat, mit wasserbasierten Oxiden von Eisen, und dann Kalzinieren bei einer Temperatur von mindestens 275°C.

4. Verfahren nach Anspruch 1, weiter umfassend die Schritte:
(a) Inkontaktbringen eines Alkohols und des Ethylens und Kohlenmonoxids mit einem Ethylen-Carbonylierungskatalysator, um ein Alkylpropionat herzustellen, und
(b) Trennen des Alkylpropionats von Nebenprodukten und Ausgangsmaterialien.

5. Verfahren nach Anspruch 4, weiter umfassend das Umsetzen des Alkylpropionats mit Formaldheyd, um ein Alkylmethacrylat herzustellen.

6. Verfahren nach Anspruch 5, in welchem der Alkohol Methanol ist, das Alkylpropionat Methylpropionat ist und das Alkylmethacrylat Methylmethacrylat ist.

7. Verfahren nach Anspruch 6, weiter umfassend das Polymerisieren des Methylmethacrylats.

8. Verfahren nach Anspruch 1, weiter umfassend das Copolymerisieren des Ethylens und Kohlenmonoxids.

9. Verfahren nach Anspruch 1, weiter umfassend das Kombinieren des Ethylens und Kohlenmonoxids mit Wasserstoff, um Propionaldehyd herzustellen.

10. Verfahren nach Anspruch 9, weiter umfassend das Kondensieren des Propionaldehyds mit Formaldehyd, um Methacrolein herzustellen.

## Revendications

1. Procédé de production d'un mélange d'éthylène et de monoxyde de carbone par mise en contact d'éthane et de dioxyde de carbone avec un catalyseur comprenant de l'oxyde de fer supporté sur un matériau inorganique comprenant au moins un parmi l'oxyde de calcium et le carbonate de calcium à une température d'au moins 600°C pour produire de l'éthylène et du monoxyde de carbone.

2. Procédé selon la revendication 1, dans lequel le catalyseur contient de 2 % à 25 % de fer.

3. Procédé selon la revendication 2, dans lequel le catalyseur est produit par revêtement de particules d'un matériau contenant au moins 50 % de carbonate de calcium avec des oxydes hydratés de fer et par calcination subséquente à une température d'au moins 275°C.

4. Procédé selon la revendication 1 comprenant de plus les étapes consistant :
(a) à mettre un alcool et lesdits éthylène et monoxyde de carbone en contact avec un catalyseur de carbonylation d'éthylène pour produire un propionate d'alkyle ; et
(b) à séparer le propionate d'alkyle des produits secondaires et des matières premières.

5. Procédé selon la revendication 4 comprenant de plus la réaction du propionate d'alkyle avec du formaldéhyde pour produire un méthacrylate d'alkyle.

6. Procédé selon la revendication 5, dans lequel l'alcool est le méthanol, le propionate d'alkyle est le propionate de méthyle et le méthacrylate d'alkyle est le méthacrylate de méthyle.

7. Procédé selon la revendication 6 comprenant de plus la polymérisation du méthacrylate de méthyle.

8. Procédé selon la revendication 1 comprenant de plus la copolymérisation de l'éthylène et du monoxyde de carbone.

9. Procédé selon la revendication 1 comprenant de plus la combinaison desdits éthylène et monoxyde de carbone avec de l'hydrogène pour produire du propionaldéhyde.

10. Procédé selon la revendication 9 comprenant de plus la condensation du propionaldéhyde avec du formaldéhyde pour produire de la méthacroléine.
